(19) Europäisches Patentamt
European Patent Office
Office européen des brevets

(11) **EP 3 097 861 B1**

(12) **EUROPEAN PATENT SPECIFICATION**

(45) Date of publication and mention
of the grant of the patent:
**06.01.2021 Bulletin 2021/01**

(51) Int Cl.:
***A61B 8/12*** *(2006.01)*

(21) Application number: **14879833.3**

(22) Date of filing: **24.10.2014**

(86) International application number:
**PCT/JP2014/078370**

(87) International publication number:
**WO 2015/111265 (30.07.2015 Gazette 2015/30)**

(54) **ULTRASONIC DIAGNOSTIC DEVICE**

ULTRASCHALLDIAGNOSEVORRICHTUNG

DISPOSITIF DE DIAGNOSTIC À ULTRASONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.01.2014 JP 2014010650**

(43) Date of publication of application:
**30.11.2016 Bulletin 2016/48**

(73) Proprietor: **Hitachi, Ltd.
Chiyoda-ku,
Tokyo 100-8280 (JP)**

(72) Inventors:
• **MOTOKI, Kazuya
Mitaka-shi
Tokyo 181-8622 (JP)**
• **WATANABE, Toru
Mitaka-shi
Tokyo 181-8622 (JP)**

(74) Representative: **Strehl Schübel-Hopf & Partner
Maximilianstrasse 54
80538 München (DE)**

(56) References cited:
JP-A- H10 118 069    JP-A- 2003 052 693
JP-A- 2003 052 693    JP-A- 2004 275 545
JP-A- 2005 253 776    JP-A- 2006 346 105
JP-A- 2009 005 994    JP-A- 2009 034 386
JP-A- 2009 261 840    JP-A- 2010 088 591
JP-A- 2011 004 874    JP-A- 2011 188 956
JP-A- 2013 052 023    US-A1- 2006 004 290
US-A1- 2011 060 225    US-A1- 2012 226 160
US-A1- 2013 006 153    US-A1- 2014 005 546

**Description**

[TECHNICAL FIELD]

The present invention relates to an ultrasonic diagnostic device, in particular to techniques to control a surface temperature of an ultrasonic-wave transmission/reception surface.

[BACKGROUND ART]

An ultrasonic diagnostic device transmits and receives ultrasonic waves to and from a subject, and forms ultrasonic wave images based on received signals. Such an ultrasonic diagnostic device includes an ultrasonic probe that transmits and receives ultrasonic waves. The ultrasonic probe includes an oscillator that transmits and receives ultrasonic waves and an electronic circuit that supplies transmission signals to the oscillator and processes received signals from the oscillator. The electronic circuit is disposed, for example, on a 3D probe that provides 3D images as ultrasonic wave images. The electronic circuit is provided for channel reduction in the ultrasonic probe so as to reduce the number of cables connecting between the ultrasonic probe and the device body, or for any other purposes. Receiving supply" " of electric power, the oscillator and the electronic circuit are activated to allow transmission and reception of ultrasonic waves. This activation of the oscillator and the electronic circuit causes them to generate heat.

As the ultrasonic probe is used in contact with a subject, the temperature of the contact surface must be appropriately controlled so as to avoid the subject from being harmed by the heat generated by the ultrasonic probe. The temperature control is particularly important for body cavity probes. The surface temperature of an ultrasonic probe is defined in regulations. For example, International Electrotechnical Commission (IEC) limits the surface temperature of a portion of the ultrasonic probe to be in contact with a patient in a normal state to be 43°C or lower.

The simplest way to control the temperature of the contact surface of the ultrasonic probe, that is, the ultrasonic-wave transmission/reception surface, is to dispose a temperature sensor on the wave transmission/reception surface. However, the temperature sensor disposed on the ultrasonic-wave transmission/reception surface may cause noise in transmitted and received waves due to influences from the temperature sensor, possibly resulting in problems such as a deterioration of ultrasonic wave images. Accordingly, disposing the temperature sensor on the wave transmission/reception surface is inappropriate.

Conventionally, the temperature of an ultrasonic probe is controlled by, for example, disposing a temperature sensor on an ultrasonic probe.

Patent Document 1 discloses techniques for predicting the temperature in the vicinity of an oscillator of an ultrasonic probe . In the invention disclosed in Patent Document 1, a temperature sensor is disposed near to an oscillator. A temperature predictor measures an output value of the temperature sensor at predetermined time intervals. The temperature predictor predicts a subsequent temperature rise based on the output value of the temperature sensor measured at the predetermined time intervals. As another method, Patent Document 1 describes to calculate the power consumption of the oscillator based on the voltage and current supplied to the oscillator, and predict a temperature rise after a predetermined time based on the calculated power consumption.

Patent Document 2 discloses that an ultrasonic oscillator is driven by a drive voltage in accordance with a table showing a relationship between a drive voltage of the ultrasonic oscillator and the maximum saturation temperature of the ultrasonic probe to avoid the maximum saturation temperature from exceeding an allowable temperature.

Patent Document 3 discloses that first and second temperature sensors are embedded in a backing material such that the surface temperature of the ultrasonic probe is estimated based on the temperatures sensed by these two temperature sensors. In Patent Document 3, the surface temperature of the ultrasonic probe is estimated based on sensed values Ta, Tb sensed by the first and second temperature sensors, a thermal resistance Ra between the first temperature sensor and the ultrasonic probe surface, and a thermal resistance Rb between the first temperature sensor and the second temperature sensor.

The document US 212/0226160 A1) discloses an ultrasonic diagnostic apparatus including a probe equipped with a temperature sensor for detecting an internal temperature of the ultrasound probe.

The document US 2014/0005546 A1 teaches to provide various temperature sensors for measuring the respective temperatures of various elements.

The document JP 2013-52693A teaches to place a temperature sensor on the inner wall of a housing of the probe and to use its signals for estimating the temperature of a wave transmission / reception surface.

[RELATED ART DOCUMENTS]

[Patent Documents]

Patent Document 1: JP 2009-034386 A
Patent Document 2: JP 2011-188956 A
Patent Document 3: JP 2009-005994 A

[DISCLOSURE OF THE INVENTION]

[Object to be Achieved by the Invention]

The invention is defined in the independent claim 1. The invention disclosed in Patent Document 1 is for controlling peripheral temperature of an oscillator. A temperature sensor is disposed to an oscillator to control peripheral temperature of the oscillator. Patent Document 1 nowhere describes that the temperature of the wave transmission/reception surface is estimated based on the peripheral temperature of the oscillator. In order to perform the temperature control of the wave transmission/reception surface based on Patent Document 1, a temperature sensor must be disposed on the wave transmission/reception surface. However, disposing a temperature sensor on the wave transmission/reception surface is inappropriate, as described above. Accordingly, the techniques disclosed in Patent Document 1 cannot be applied without modification to measure the temperature of the wave transmission/reception surface.

In the invention disclosed in Patent Document 2, the drive voltage of an ultrasonic probe is controlled based on the saturation temperature of the probe surface, that is, the temperature of the probe surface when the temperature of the probe surface reaches thermal equilibrium at a certain period of time after the start of the transmission/reception of ultrasonic waves. Accordingly, the temperature of the probe surface is not controlled in real time. In the invention disclosed in Patent Document 2, the ultrasonic probe cannot be driven by the drive voltage which is expected to cause the maximum saturation temperature to exceed an allowable temperature. Accordingly, flexible usages such as selecting operation conditions for high quality images only in a short period of time cannot be applied.

In the invention described in Patent Document 3, sensed temperature values and thermal resistances are taken into consideration, and a part of the heat generated by a heat source is recognized as a quantity of heat flow. However, as a heat source is disposed away from the temperature sensor in the invention disclosed in Patent Document 3, thermal responsiveness is expected to be low, and thus the accuracy in estimating the surface temperature of the ultrasonic probe may be influenced. In addition, as the temperature sensor is disposed on the center axis of the ultrasonic probe, a temperature distribution cannot be sensed.

An object of the present invention is to accurately estimate the surface temperature of a wave transmission/reception surface of an ultrasonic probe.

[Means for Achieving the Object]

(1) The present invention relates to an ultrasonic diagnostic device. The ultrasonic diagnostic device is characterized by including an ultrasonic probe that includes a wave transceiver unit including an oscillator that transmits and receives ultrasonic waves, a wave transmission/reception surface disposed on a living subject side of the oscillator, and an electronic circuit connected to the oscillator, and a temperature sensing unit disposed in the wave transceiver unit, and a temperature estimation unit that estimates a surface temperature of the wave transmission/reception surface based on a wave transmission/reception condition of the ultrasonic waves, and a sensed value sensed by the temperature sensing unit.

According to the above configuration, the temperature sensing unit is disposed in the wave transceiver unit of the ultrasonic probe. As described above, disposing the temperature sensing unit on the wave transmission/reception surface is inappropriate. Accordingly, in the above configuration, the temperature sensing unit is disposed in the wave transceiver unit. As the temperature sensing unit does not directly sense the surface temperature of the wave transmission/reception surface of the ultrasonic probe, the temperature of the wave transmission/reception surface must be estimated based on the sensed value from the temperature sensing unit. As the temperature of the wave transmission/reception surface corresponding to the sensed value from the temperature sensing unit varies depending on a transmission/reception condition of ultrasonic waves, the temperature of the wave transmission/reception surface is estimated based on the sensed value from the temperature sensing unit in consideration of the transmission/reception condition of ultrasonic waves in the above configuration.

(2) In the above configuration (1), the temperature · estimation unit preferably includes a temperature difference estimation unit that estimates a difference between the temperature at a temperature sensing position of the temperature sensing unit and the temperature of the wave transmission/reception surface based on the wave transmission/reception condition of ultrasonic waves, and a surface temperature estimator that estimates the surface temperature based on the temperature difference and the sensed value from the temperature sensing unit.

(3) In the above configuration (1), the temperature difference estimation unit preferably estimates the temperature difference based on power consumption for wave transmission/reception calculated in accordance with the wave transmission/reception condition of ultrasonic waves. As the difference between the temperature at the temperature sensing position of the temperature sensing unit and the temperature of the wave transmission/reception surface varies in accordance with the power consumption of a heat source, the accuracy in temperature estimation of wave transmission/reception surface is improved by considering the power consumption. Further, the power consumption for wave transmission/reception preferably includes a first power consumption at the electronic circuit and a second power consumption at the oscillator. When two or more heat sources are provided, the estimation accuracy of surface temperature can be further improved by individually considering the power consumptions of respective heat sources.

(4) In the above configuration (1), the wave transceiver unit further includes a relay board sandwiched between the electronic circuit and the oscillator, and the temperature sensing unit is disposed on the relay board.

In the above described configuration, the relay board is provided on a side on which the wave transmission/reception surface is provided when viewed from the electronic circuit, such that the heat generated from the electronic circuit is transmitted to the wave transmission/reception surface via the relay board. By providing the temperature sensing unit on this relay board, the heat generated from the electronic circuit which would cause a temperature rise of the wave transmission/reception surface can be sensed directly or highly responsively. Naturally, it is also possible to dispose the temperature sensing unit in the electronic circuit itself. When the electronic circuit generates much more heat than the oscillator, in other words, when the electronic circuit is the main heat source, the above configuration can improve accuracy in the estimation of the surface temperature. If required, a second temperature sensing unit may be disposed in the vicinity of the oscillator to additionally consider the sensed value of the second temperature sensing unit. The above configuration allows highly accurate positioning of the temperature sensing unit in the case where the relay board is formed as a rigid substrate.

(5) In the above configuration (4), the temperature sensing unit is preferably disposed, in the vicinity of the electronic circuit, on a side of the relay board on which the electronic circuit is disposed.

According to the above configuration, a change in the quantity of generated heat can be promptly sensed by disposing the temperature sensing unit in the vicinity of the main heat source when the main heat source of the ultrasonic probe is the electronic circuit.

(6) In the above configuration (5), the temperature sensing unit preferably includes two or more temperature sensors disposed on the relay board. The temperature estimation unit preferably defines a reference temperature based on two or more temperatures sensed by the two or more temperature sensors, and estimates the surface temperature based on the reference temperature. The two or more temperature sensors are preferably disposed on at least right and left sides of the electronic circuit on the relay board. It is further preferable that the temperature estimation unit defines, as the reference temperature, the highest temperature among the two or more temperatures.

 According to the above configuration, the temperature sensing unit includes two or more temperature sensors . The surface temperature is estimated based on the reference temperature which is defined based on two or more sensed values. This allows temperature control based on the highest surface temperature, for example, when a temperature gradient occurs in the surface temperature.

(7) In the above configuration (1), the ultrasonic probe is preferably a probe to be inserted into a body cavity. The oscillator is preferably a 2D array oscillator. The electronic circuit is preferably a transceiver circuit having a channel reduction function that supplies two or more transmission signals to the 2D array oscillator and processes two or more reception signals from the 2D array oscillator.

(8) In the above configuration (1), the ultrasonic diagnostic device preferablly further includes a controller that controls the wave transmission/reception condition of ultrasonic waves to lower the surface temperature of the wave transmission/reception surface based on the surface temperature of the wave transmission/reception surface.

[Advantages of the Invention]

According to the present invention, the surface temperature of a wave transmission/reception surface of an ultrasonic probe can be accurately estimated.

[BRIEF DESCRIPTION OF THE DRAWINGS]

FIG. 1 is a configuration block diagram of an ultrasonic wave diagnostic device according to an embodiment of the present invention.
FIG. 2 is a cross-sectional view showing a structure of a transesophageal probe according to an embodiment of the present invention.
FIG. 3A is a plan view of a relay board.
FIG. 3B is a plan view of another relay board.
FIG. 3C is a plan view of yet another relay board.
FIG. 4 is a diagram showing a thermal circuit.
FIG. 5 is a flowchart showing an order of operations of an ultrasonic wave diagnostic device according to an embodiment of the present invention.
FIG. 6 is a graph showing estimated surface temperatures and actually measured surface temperatures.

[BEST MODE FOR CARRYING OUT THE INVENTION]

Embodiments of an ultrasonic wave diagnostic device according to the present invention are described below. It should be noted that the present invention is not limited to the embodiments described below.

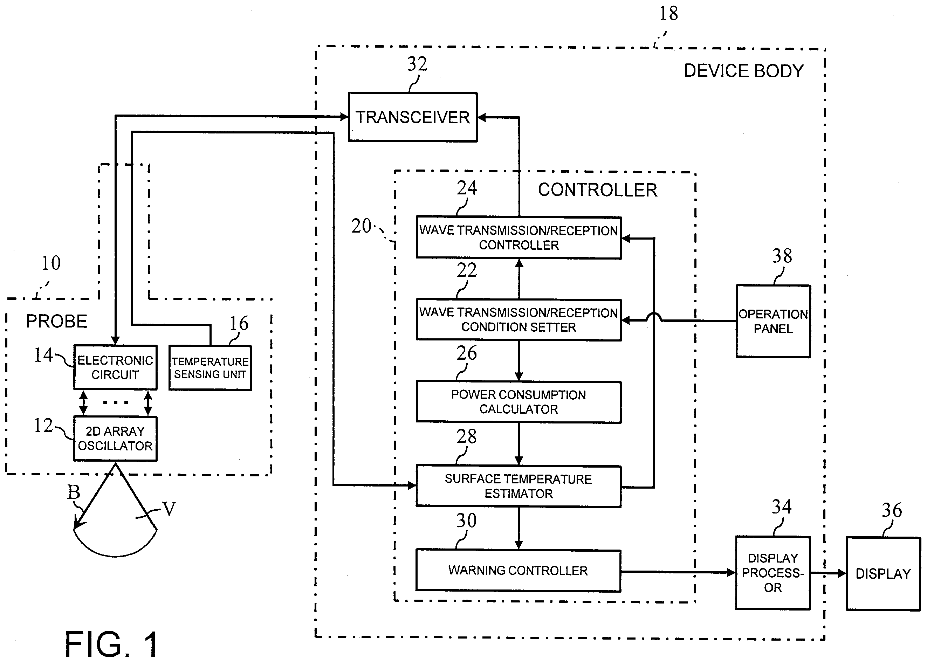

FIG. 1 is a configuration block diagram of an ultrasonic wave diagnostic device according to an embodiment of the present invention. Ultrasonic wave diagnostic devices are generally installed at medical facilities such as hospitals. The ultrasonic wave diagnostic devices are medical devices to perform ultrasonic wave diagnosis for living bodies.
A probe 10 is an ultrasonic probe which is in contact with a body surface of a subject for transmitting and receiving ultrasonic waves . The probe 10 is configured to include a 2D array oscillator 12 which transmits and receives ultrasonic waves, an electronic circuit 14 which is electrically connected to the 2D array oscillator 12, and a temperature sensing unit 16 which senses the temperature in the probe 10 at a predetermined position. The probe 10 is connected to a device body 18 described below via a cable or wirelessly. Although the probe 10 in the present embodiment is a transesophageal probe, the probe 10 may be another type, such as a convex or linear type.
The 2D array oscillator 12 includes over a thousand oscillation elements arranged in two dimensions. Using supply of drive voltage, each oscillation element forms an ultrasonic beam. Each oscillation element also receives reflection echoes reflected from the subject. Signals received by each oscillation element are sent to the electronic circuit 14. Receiving supply of electric power, the 2D array oscillator 12 is activated and generates heat. The power consumption and the quantity of generated heat at the 2D array oscillator 12 vary depending on wave transmission/reception conditions, such as a diagnostic mode, transmission voltage, wavenumber, pulse repetition time (PRT), and frequency.
The electronic circuit 14 is configured to include an application specific integrated circuit (ASIC) in which two or more circuits having different features are combined. The ASIC in the present embodiment serves as a sub transmission beam former and a sub reception beam former. The oscillation elements of the 2D array oscillator 12 are grouped in two or more groups. The sub transmission beam former generates transmission signals that have a delay between them for each group based on the group transmission signals. The sub reception beam former performs phasing addition for the reception signals for each group to generate group reception signals. The group reception signals are processed by a transceiver 32 inside the device body 18 to form a single piece of beam data. These processes reduce the number of signal wires between the probe 10 and the device body 18. Receiving supply of electric power, the ASIC is activated and generates heat. Similarly as the 2D array oscillator 12, the power consumption and the quantity of generated heat at the ASIC vary depending on transmission/reception conditions of ultrasonic waves. In the present embodiment, the quantity of generated heat at the ASIC is about 10 times the quantity of generated heat at the 2D array oscillator 12. Thus, the main heat source of the probe 10 is the ASIC.
The temperature sensing unit 16 is a temperature sensor disposed in the probe 10. The temperature sensor senses a peripheral temperature around the disposed position. In the present embodiment, a thermistor, whose resistance depends on the temperature, is used as the temperature sensor. The temperatures are sensed by obtaining the resistance of the thermistor and referring to the temperature characteristics of the thermistor. As described above, although the simplest way to measure the temperature of the wave transmission/reception surface of the probe is to dispose a temperature sensor on the wave transmission/reception surface, the temperature sensor affects the transmission/reception signals, deteriorating the ultrasonic images as a result. Accordingly, in the present embodiment, a temperature sensor is disposed at a wave transceiver unit that includes the 2D array oscillator 12 and the electronic circuit 14. The temperature sensor is disposed on a relay board disposed between the 2D array oscillator 12 and the electronic circuit 14. In this way, because the temperature sensor can be positioned nearer to the two heat sources, the heat from these heat sources can be sensed in a timely manner, enhancing the responsiveness of the temperature control. Further, by disposing the thermistor, which serves as the temperature sensor, on a substrate, the positional accuracy of the temperature sensor can be improved.

The device body 18 is configured to include a controller 20, a transceiver 32, a display processor 34, and an operation panel 38. The controller 20 includes a wave transmission/reception condition setter 22, a wave transmission/reception controller 24, a power consumption calculator 26, a surface temperature estimator 28, and a warning controller 30. These features are achieved by programs, which are stored in a storage (not shown) disposed in the device body 18.

The wave transmission/reception condition setter 22 sets transmission/reception conditions of ultrasonic waves at the 2D array oscillator 12. The wave transmission/reception conditions are set in accordance with instructions from an operator. For example, from the operation panel 38, an operator selects a desired heat generation mode from two or more heat generation modes which are provided in advance . The present embodiment offers three modes, "Low", "Mid", and "High". The heat generation mode "High" can achieve the maximum performance of the ultrasonic diagnostic device such that ultrasonic images having a high resolution and a high responsiveness can be captured. In contrast, although the image quality of the ultrasonic wave images is lowered, the heat generation mode "Low", which is achieved by lowering the transmission voltage and the wave number of the ultrasonic waves, or by increasing PRT, can achieve a longer period of operation time because of a suppressed quantity of generated heat. The heat generation mode "Mid" is an intermediate mode of these two modes. The wave transmission/reception condition setter 22 sets wave transmission/reception conditions in accordance with the selected heat generation mode. The wave transmission/reception conditions include conditions such as the drive voltage of the oscillation element, frequency, wave number, pulse repetition time (PRT), and diagnostic mode.

The wave transmission/reception controller 24 controls the transceiver 32 based on the wave transmission/reception conditions set by the wave transmission/reception condition setter 22 so as to operate the 2D array oscillator 12 and the electronic circuit 14 under the set wave transmission/reception conditions. The wave transmission/reception controller 24 also controls the transceiver 32 in accordance with signals from the wave transmission/reception condition setter 22 or the surface temperature estimator 28. Specifically, in response to receiving information indicating that the surface temperature of the probe 10 has reached a predetermined temperature (for example, 43°C), the wave transmission/reception controller 24 controls the transceiver 32 to immediately stop the transmission and the reception of ultrasonic waves.

The power consumption calculator 26 calculates power consumption of the 2D array oscillator 12 and the electronic circuit 14 based on the wave transmission/reception conditions set by the wave transmission/reception condition setter 22. The power consumption may be calculated using a function representing a relationship between the wave transmission/reception conditions and the power consumption. Alternatively, the power consumption may be specified based on the wave transmission/reception conditions and a table which can be stored in advance to represent a relationship between the wave transmission/reception conditions and power consumption.

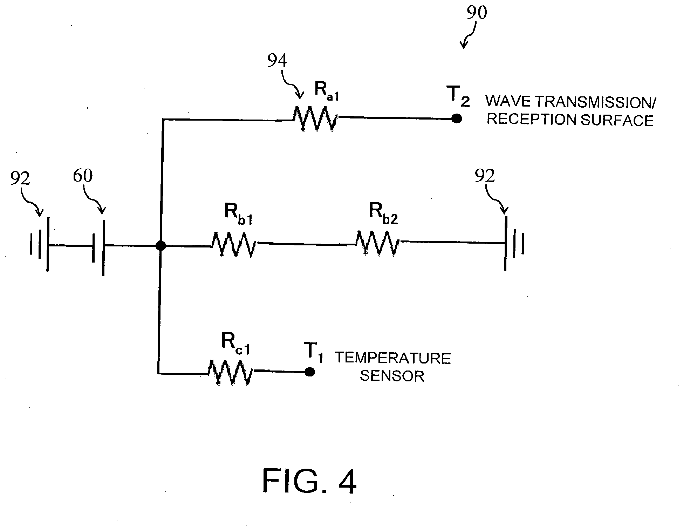

The surface temperature estimator 28 estimates the temperature of the wave transmission/reception surface of the probe 10 based on the power consumption calculated by the power consumption calculator 26 and the temperature sensed by the temperature sensing unit 16. How the temperature of the wave transmission/reception surface is estimated is described in detail below with reference to FIG. 4.

The warning controller 30 controls to output a warning to prompt the operator to lower the temperature of the transmission/reception surface based on the temperature of the wave transmission/reception surface estimated by the surface temperature estimator 28. In the present embodiment, a warning is output when the temperature of the wave transmission/reception surface is at a predetermined value (for example, 41°C) or higher. The warning may be displayed on a display 36 as a warning message, or issued as sound or light. Obviously, a combination of these means is also possible.

The transceiver 32 serves as a main transmission/reception beam former. The transceiver 32 receives signals from the wave transmission/reception controller 24 and supplies the electronic circuit 14 with signals to drive the oscillation elements of the 2D array oscillator 12. The transceiver 32 also receives reception signals from the 2D array oscillator 12 via the electronic circuit 14. The reception signals are sent to an ultrasonic image former (not shown) where ultrasonic images are formed based on the received signals.

The display processor 34 controls to display, on the display 36, the temperature of the wave transmission/reception surface of the probe 10 estimated by the surface temperature estimator 28. The displayof the surface temperature is preferably performed in real time. In response to instructions from the warning controller 30, the display processor 34 also controls to display a warning message on the display 36. The display processor 34 further controls to display, on the display 36, ultrasonic images formed by the ultrasonic image former.

It should be noted that among the elements shown in FIG. 1, the transceiver 32, the elements included in the controller 20, and the display processor 34 may be achieved by hardware such as electrical and electronic circuits and processors. Additional devices such as a memory may be used as required to provide the elements. Further, the features of the above elements may be achieved by a combination of hardware such as a CPU, a processor, and a memory, and software (program) which defines operations of the CPU and the processor. A preferable embodiment of the display 36 is a liquid crystal display.

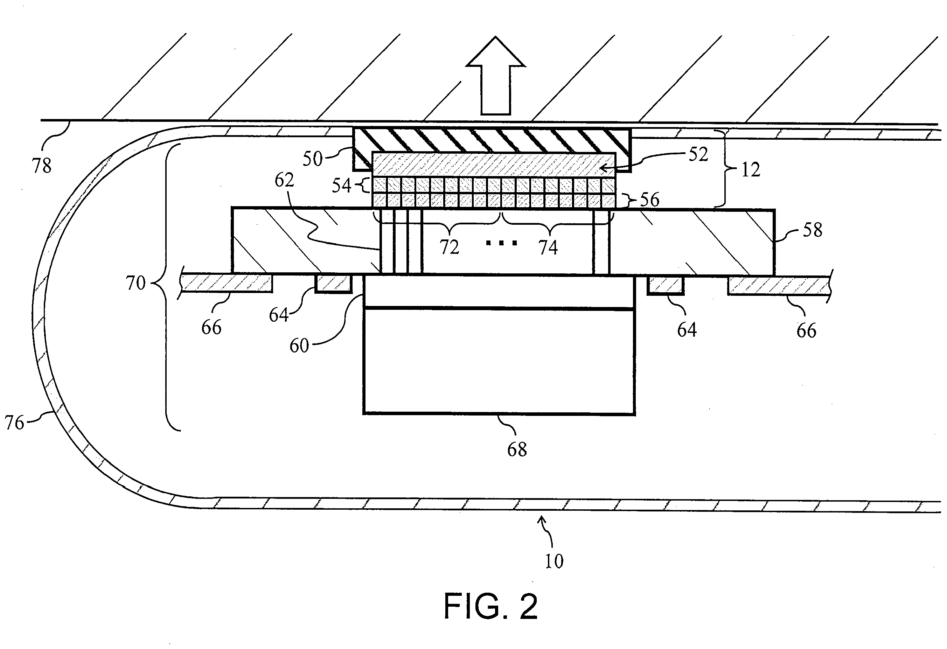

FIG. 2 is a cross-sectional view showing a structure of a transesophageal probe according to an embodiment of the present invention. An ultrasonic wave transceiver unit 70 includes elements shown in FIG. 2 such as a protection layer 50, an acoustic matching layer 52, oscillation elements 54, backing members 56, a relay board 58, an ASIC 60, a FPC 66, a heatsink member 68, etc.

The protection layer 50 protects the acoustic matching layer 52 and the succeeding layers. The protection layer 50 may be made of silicon rubber and curved. As the acoustic matching layer 52 and the oscillation elements 54 are disposed under the protection layer 50, ultrasonic waves are transmitted and received through the protection layer 50. Accordingly, the surface on the subject 78 side of the protection layer 50 is the wave transmission/reception surface.

The acoustic matching layer 52 functions to match the acoustic impedance between the oscillation elements 54 and the subject 78 to suppress the reflection of ultrasonic waves. The acoustic matching layer 52 may include one or more layers. The acoustic matching layer 52 realizes preferable ultrasonic images.

The oscillation elements 54 are used to form ultrasonic wave beams or reception signals. The oscillation elements 54 contain ceramic such as lead zirconate titanate (PZT) or a single crystal such as lead magnesium niobate-lead titanate (PMT-PT) solid solution. As described above, over a thousand oscillation elements 54 are disposed . An electrode through which drive voltage is applied is attached to both sides of each oscillation element 54. With the drive voltage applied, the oscillation elements 54 oscillates by repeating contraction and expansion to generate ultrasonic beams. Further, the oscillation elements 54 receive reflected echoes from the subject and generate voltage by being oscillated by the reflected echoes. This forms reception signals.

The backing members 56 function to suppress unnecessary oscillations of the oscillation elements 54. Each of the backing members 56 is provided for each of the oscillation elements 54. The backing members 56 preferably contain a hard backing (HB) material having high acoustic impedance.

The 2D array oscillator 12 includes the protection layer 50, the acoustic matching layer 52, the oscillation elements 54, and the backing members 56.

The relay board 58 is disposed between the 2D array oscillator 12 and the ASIC 60, which forms the electronic circuit 14. In order to electrically connect between the two, the relay board 58 includes two or more lead wires 62. The subject side of the relay board 58 is electrically connected to the 2D array oscillator 12, whereas the opposite side (non-subject side) of the relay board 58 is connected to the ASIC 60. The oscillation elements 54 and the ASIC 60 are electrically connected to each other by the lead wires 62. The relay board 58 is preferably rigid to an extent that no deformation is caused with the probe 10 in normal use. By providing the temperature sensor on the rigid relay board 58, the positional accuracy of the temperature sensor can be improved.

The relay board 58 includes a thermistor 64, which is a temperature sensor. In the present embodiment, two thermistors are provided. The thermistors 64 are preferably provided on the non-subject side of the relay board 58, in other words, the same side as the ASIC 60. In particular, disposing the thermistors 64 near to the ASIC 60 allows earlier sensing of a change in the quantity of generated heat of the ASIC 60. Further, radiation of heat which is generated from the ASIC 60 and transferred through the air can also be sensed. The thermistors 64 may be mounted on the relay board 58 using flip chip mounting or reflow mounting.

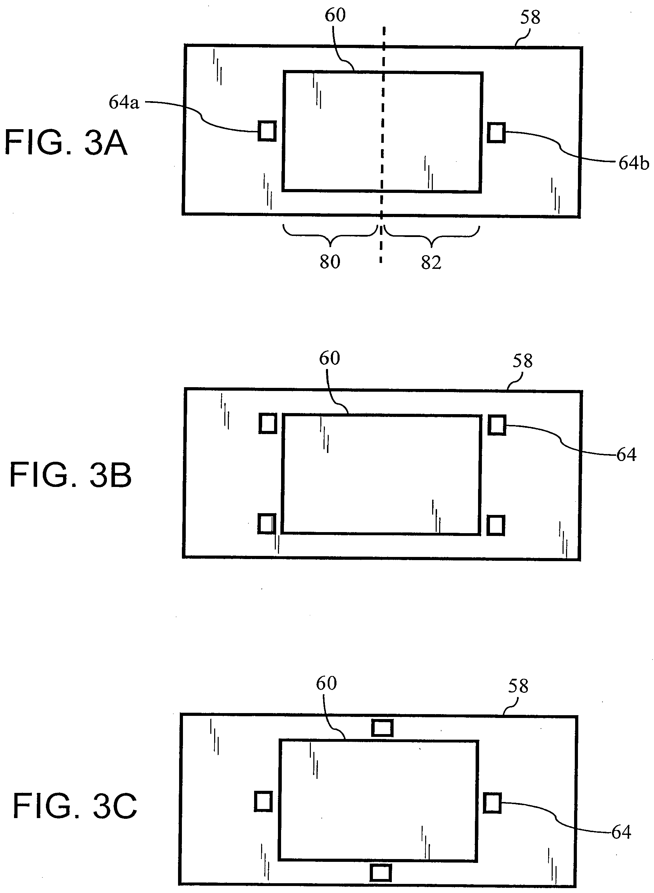

It is preferable that two ormore thermistors 64 are provided. The thermistors 64 disposed at several locations allow sensing temperatures at two or more locations of the probe 10. For example, in a continuous wave (CW) Doppler system, in which the transmission and the reception of ultrasonic waves are performed by respective oscillation elements 54, the quantity of generated heat of the oscillation elements 54 for transmission may be different from that for reception. Further, the quantity of generated heat may also be different between the portion of the ASIC used for transmission corresponding to the oscillation elements for transmission and the portion of the ASIC used for reception corresponding to the oscillation elements for reception. This difference in the quantities of generated heat may cause a temperature gradient on the wave transmission/reception surface. In such a case, an occurrence of the temperature gradient on the wave transmission/reception surface can be assumed based on the temperatures sensed at two or more locations. Accordingly, the transmission and reception of the ultrasonic waves can be controlled using a higher temperature of the wave transmission/reception surface. The positions of the thermistors 64 are described below with reference to FIGs 3A to 3C.

The flexible printed circuit (FPC) 66 is used to connect between the device body 18 and other elements such as the oscillation elements 54, the ASIC 60, and the thermistor 64. The FPC 66 is electrically connected to the relay board 58 such that the FPC 66 is connected to the elements such as the oscillation elements 54 via the relay board 58.

The heatsink member 68 is disposed in contact with the ASIC 60. The heatsink member 68 is used to dissipate heat generated from the ASIC 60 into the air or to the other elements such as a chassis 76. The heatsink member 68 may also have a function to absorb ultrasonic waves which have propagated to the surface opposite to the reflection surface.

The chassis 76 is a chassis of the probe 10. As the probe 10 in the present embodiment is a transesophageal probe, the tip is bullet-shaped for easy insertion into the esophagus of a patient.

FIGs. 3A, 3B, and 3C show plan views of the relay boards. These drawings show arrangement examples of the ASIC 60 and the thermistors 64 on the relay board 58. In the example shown in FIG. 3A, the ASIC 60 and the thermistors 64 are arranged in a line along the longitudinal direction of the relay board 58 such that the thermistors 64 are arranged on both sides of the ASIC 60.

For the ultrasonic wave transmission/reception in the CW Doppler system, the oscillation elements 54 are divided into two groups, one group for transmission and the other for reception. For example, in FIG. 2, a half of the oscillation elements 54 on the left are used as transmission oscillation elements 72 and the other half on the right are used as reception oscillation elements 74. In such a case, as shown in FIG. 3A, the ASIC 60 is divided into a transmission portion 80 and a reception portion 82 respectively corresponding to the transmission oscillation elements 72 and the reception oscillation elements 74. A difference in the quantity of generated heat may be caused between the two elements. For example, the quantity of generated heat of the transmission oscillation elements 72 is larger than that of the reception oscillation elements 74, and correspondingly, the quantity of generated heat of the transmission portion 80 is larger than that of the reception portion 82. Such a difference in the quantities of generated heat causes a temperature gradient on the wave transmission/reception surface of the probe 10.

In this situation, the temperature sensed by the thermistor 64a in FIG. 3A is higher than the temperature sensed by the thermistor 64b. Therefore, based on this difference between the sensed temperatures of these two thermistors 64a, 64b, a temperature gradient can be assumed. For example, by defining, as a reference temperature, the highest sensed temperature value from the two or more thermistors 64, the surface temperature of the wave transmission/reception surface of the probe 10 can be calculated based on this reference temperature. This allows appropriate control, for example, by stopping transmission and reception of ultrasonic waves, even when only surface temperatures at some locations on the wave transmission/reception surface are higher than a predetermined temperature.

FIGs. 3B and 3C show other arrangement examples of the thermistors 64. Although four thermistors 64 are arranged in FIGs . 3B and 3C, more thermistors 64 may be disposed. If more thermistors 64 are provided, a more detailed recognition of temperature gradient becomes possible. The positions of the thermistors 64 may be determined based on the positions of the transmission portion 80 and the reception portion 82 of the ASIC in the CW Doppler system.

As a method of determining the reference temperature when two or more thermistors 64 are disposed, other than setting the highest sensed temperature as described above, it is also possible to set the reference temperature by disposing two or more thermistors 64 near to each other as a thermistor group and setting an average of sensed temperature values from respective thermistors as the reference temperature. This can reduce influences to each thermistor caused by the differences in the sensed temperatures. With two or more thermistor groups provided, the highest temperature among the average temperatures of respective thermistor groups may also be set as the reference temperature.

FIG. 4 shows a thermal network. As the relationships among a temperature difference between objects, a thermal flow, and a thermal resistance are similar to those among a voltage, a current, and a resistance in an electric circuit, the temperature difference, the thermal flow, and the thermal resistance can be represented in a thermal network, which is similar to an electronic circuit. The thermal network 90 includes a reference temperature 92, which is represented by the same symbol as a ground in an electric circuit, a heat source, which is represented by the same symbol as a DC power supply in an electric circuit, and a thermal resistance 94, which is represented by the same symbol as a resistance in an electric circuit. For simplicity, the thermal network 90 is assumed to include a single heat source.

In the thermal network 90, an outside air temperature is set as the reference temperature 92. The heat source is the ASIC 60 in FIG. 2. The thermal resistance 94 represents a measure of difficulty of heat transfer through each element in the probe 10 in the unit of "°C/W". For example, the thermal resistance $R_{a1}$ between the ASIC 60 and the wave transmission/reception surface represents a combined resistance of thermal resistances of the elements between the ASIC 60 and the wave transmission/reception surface, such as the relay board 58, the lead wires 62, the backing members 56, the acoustic matching layer 52, and the protection layer 50.

How to estimate the temperature of the wave transmission/reception surface of the probe 10 by the surface temperature estimator 28 is described below with reference to FIGs. 1 and 4. The surface temperature estimator 28 estimates the temperature of the wave transmission/reception surface of the probe 10 based on the following equations:

Equation 1

(Wave transmission/reception surface temperature $T_2$) = (Temperature sensor sensed temperature $T_1$) + (Difference between temperature at temperature sensing position of temperature sensor and temperature of wave transmission/reception surface) + (Primary delay factor)

Equation 2

(Difference between temperature at temperature sensing position of temperature sensor and temperature of wave transmission/reception surface) = $\alpha$ x power consumption W

Equation 3

(Primary delay factor) = $\beta$ x exp $(-\Delta t/K)$

In Equation 1, "(Difference between temperature at temperature sensing position of temperature sensor and temperature of wave transmission/reception surface)" represents a difference 15 between the temperature at the disposed position of the thermistor 64 and the temperature of the wave transmission/reception surface, which is estimated by Equation 2. In addition, "(Primary delay factor)" is added in consideration of a time difference required for the heat to reach the wave transmission/reception surface from the heat source.

"$\alpha$" in Equation 2 represents a value obtained by subtracting the thermal resistance between the heat source and the wave transmission/reception surface from the thermal resistance between the heat source and the temperature sensor. For example, assuming that, in the thermal network 90, the thermal resistance $R_{c1}$ between the heat source, ASIC 60, and the temperature sensor is 2°C/W, whereas the thermal resistance $R_{a1}$ between the ASIC 60 and the wave transmission/reception surface is 6°C/W, $\alpha$ becomes "2 - 6 = -4". The power consumption W in Equation 2 represents the power consumption of the heat source, ASIC 60. Assuming that the power consumption of the ASIC 60 is 1W, the difference between the temperature at the disposed position of the temperature sensor (the thermistor 64) and the temperature of the wave transmission/reception surface can be estimated by -4 x 1 = -4°C.

"$\beta$" in Equation 3 represents a variable which varies in accordance with the power consumption of the heat source. Further, "$\Delta t$" represents an elapsed time (sec) after the start of transmission/reception of ultrasonic waves, and "$\kappa$" represents a time constant.

When two or more heat sources are provided, the differences between temperature at a temperature sensing position of a temperature sensor and temperature of a wave transmission/reception surface are calculated for respective heat sources and the total value obtained by adding the calculated values for respective heat sources are used as the value for the term " (difference between temperature at temperature sensing position of temperature sensor and temperature of wave transmission/reception surface)" in Equation 1. For example, assuming that two heat sources (the ASIC 60 and the oscillation elements oscillation elements 54) are provided, first, a value "$\alpha_1$" is obtained by subtracting the thermal resistance between the ASIC 60 and the wave transmission/reception surface from the thermal resistance between the ASIC 60 and the temperature sensor to further obtain a value $\alpha_1 W_1$ by multiplying $\alpha_1$ by the power consumption $W_1$ of the ASIC 60. Next, a value "$\alpha_2$" is obtained by subtracting the thermal resistance between the oscillation elements 54 and the wave transmission/reception surface from the thermal resistance between the oscillation elements 54 and the temperature sensor to further obtain a value $\alpha_2 W_2$ by multiplying $\alpha_2$ by the power consumption $W_2$ of the oscillation elements 54. Then, a value $\alpha_1 W_1 + \alpha_2 W_2$ can be used as the term " (Difference between temperature at temperature sensing position of temperature sensor and temperature of wave transmission/reception surface)" in Equation 1.

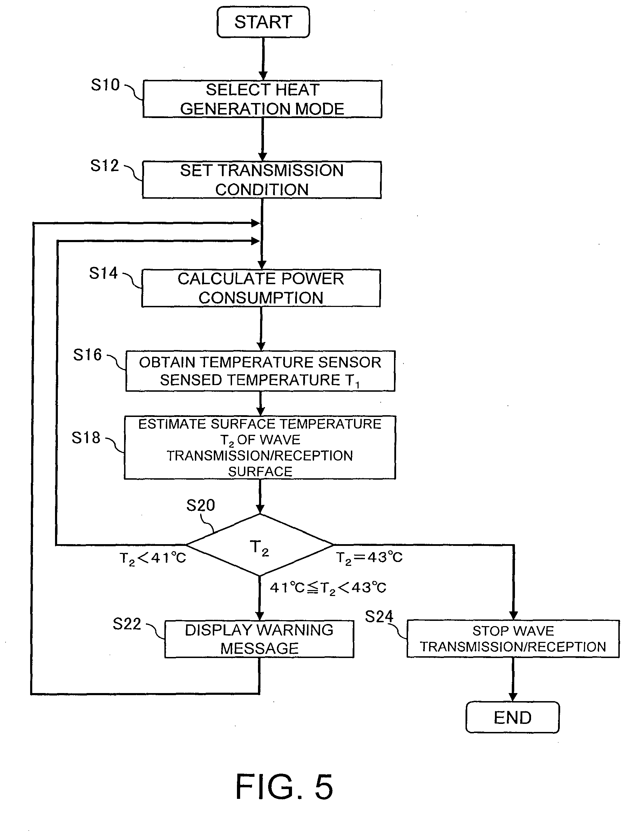

FIG. 5 is a flow chart showing operations of the controller of the ultrasonic diagnostic device according to an embodiment of the present invention. The operation flow of the controller of the ultrasonic diagnostic device according to the present embodiment is described below with reference to FIGs. 1, 2, and 5.

In step S10, an operator selects a heat generation mode. The operator selects one of the heat generation modes , "Low", "Mid", and "High" by operating the operation panel 38.

In step S12, the wave transmission/reception condition setter 22 sets transmission/reception conditions of ultrasonic waves in accordance with the heat generation mode input by the operator.

In step S14, the power consumption calculator 26 calculates the power consumption of the oscillation elements 54 and the ASIC 60 in accordance with the transmission/reception conditions of the ultrasonic waves set in step S12.

In step S16, the surface temperature estimator 28 obtains a temperature $T_1$ sensed by the temperature sensor. Specifically, the surface temperature estimator 28 reads the resistance of the thermistor 64 and obtains, as the sensed temperature $T_1$, the temperature corresponding to the read resistance based on temperature characteristics of the thermistor 64.

In step S18, the surface temperature estimator 28 estimates a surface temperature $T_2$ of the wave transmission/reception surface . As described above, the surface temperature $T_2$ is estimated based on the sensed temperature $T_1$ sensed by the temperature sensor and the power consumptions of the heat sources, namely, the oscillation elements 54 and the ASIC 60.

In step S20, the surface temperature estimator 28 determines whether or not the estimated surface temperature $T_2$ is higher than a first threshold and a second threshold. In the present embodiment, no control is performed when the surface temperature $T_2$ is lower than the first threshold. When the surface temperature $T_2$ is equal to or higher than the first threshold but lower than the second threshold, a warning is issued. When the surface temperature $T_2$ has reached the second threshold, control is performed to immediately stop the transmission/reception of ultrasonic waves. While the first and second thresholds may be set at any value, in view of IEC standards, the first threshold is set at 41°C and the second threshold is set at 43°C in the present embodiment.

In step S20, when the surface temperature $T_2$ is lower than the first threshold, the process returns to step S14 and repeats steps S14 to S20. When the surface temperature $T_2$ is equal to or higher than the first threshold but lower than the second threshold, the process proceeds to step S22.

In step S22, the warning controller 30 sends instructions to the display processor 34 to display a warning message on the display 36. For example, the warning message may include the surface temperature $T_2$ estimated in step S18, a message indicating that the estimated temperature $T_2$ is close to the second threshold at which the transmission/reception of ultrasonic waves is stopped, and a message to prompt the operator to switch the heat generation mode to a lower mode. After displaying the warning message in step S22, the processes in steps S14 to S20 are performed again.

In step S20, when the surface temperature $T_2$ has reached the second threshold, the process proceeds to step S24. In step S24, the wave transmission/reception controller 24 controls the transceiver 32 to immediately stop the transmission/reception of ultrasonic waves. At the same time, a message may also be displayed on the display 36, indicating that the transmission/reception of ultrasonic waves is stopped because the surface temperature $T_2$ has reached the second threshold.

As described above, as the estimation of the surface temperature $T_2$ of the wave transmission/reception surface is repeatedly performed, the estimated value of the surface temperature $T_2$ can be obtained in real time. This allows flexible applications, such as using an operation mode, which would normally cause the surface temperature $T_2$ to reach the temperature limit if used for a long period, for only a short period Further, because the transmission/reception of ultrasonic waves is forced to be stopped when the surface temperature $T_2$ reaches the temperature limit and a warning is issued when the surface temperature $T_2$ is close to the temperature limit, damage to the subject due to careless operation by the operator can be avoided.

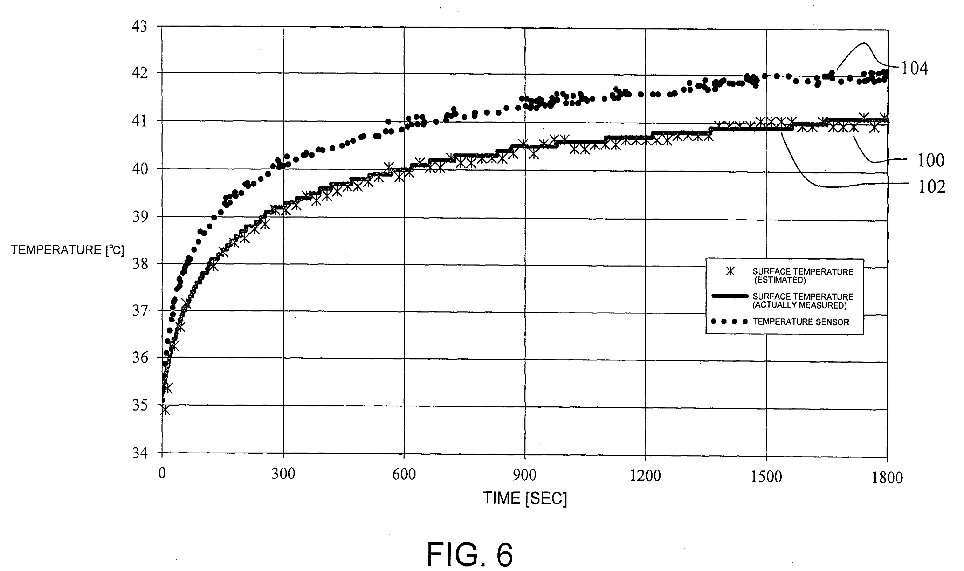

FIG. 6 shows a graph showing estimated surface temperatures and actually measured surface temperatures. The curve 100 (* symbols) represents temperatures of the wave transmission/reception surface estimated by the surface temperature estimator 28. The curve 102 (bold line) represents actually measured temperatures of the wave transmission/reception surface. The curve 104 (dots) represents temperatures sensed by the temperature sensor. As is apparent from the comparison between the curve 100 and the curve 102, the present invention can achieve accurate estimation of the temperature of the wave transmission/reception surface.

[REFERENCE NUMERALS]

10 probe, 12 2D array oscillator, 14 electronic circuit, 16 temperature sensing unit, 18 device body, 20 controller, 22 wave transmission/reception condition setter, 24 wave transmission/reception controller, 26 power consumption calculator, 28 surface temperature estimator, 30 warning controller, 32 transceiver, 34 display processor, 36 display, 38 operation panel, 50 protection layer, 52 acoustic matching layer, 54 oscillation elements, 56 backing members, 58 relay board, 60 ASIC, 62 lead wires, 64, 64a, 64b thermistors, 66 FPC, 68 heatsink member, 70 wave transceiver unit, 72 transmission oscillation elements, 74 reception oscillation elements, 76chassis, 78 subject, 80 transmission portion, 82 reception portion, 90 thermal network, 92 reference temperature, 94 thermal resistance, and 100, 102, 104 curves.

**Claims**

1. An ultrasonic diagnostic device comprising:

   an ultrasonic probe (10) comprising a wave transceiver unit (70) including an oscillator (54) that transmits and receives ultrasonic waves, a wave transmission/reception surface of the oscillator (54) configured to be disposed on a living subject side, a relay board which is formed as a rigid substrate (58), and an electronic circuit (60) electrically connected to the oscillator (54) and
   a temperature sensing unit (64) disposed in the wave transceiver unit;
   wherein
   the rigid substrate (58) is sandwiched between the electronic circuit and the oscillator (54), and
   the temperature sensing unit (64) is disposed on the rigid substrate (58) and wherein
   the ultrasonic diagnostic device
   further comprises a temperature estimation unit (28) configured to estimate, for each of at least two wave transmission/reception conditions of the ultrasonic waves, a surface temperature of the wave transmission/reception surface based on:

   • the wave transmission/reception condition of the ultrasonic waves, and
   • a sensed value sensed by the temperature sensing unit (64)

   wherein the temperature estimation unit (28) is further configured to add a primary delay factor in consideration of a time difference required for the heat to reach the wave transmission/reception surface from the heat source when estimating the surface temperature of the wave transmission/reception surface.

2. The ultrasonic diagnostic device according to claim 1,
   wherein
   the temperature estimation unit (28) comprises
   a temperature difference estimation unit that estimates a difference between the temperature at a temperature sensing position of the temperature sensing unit (64) and the temperature of the wave transmission/reception surface based on the wave transmission/reception condition of the ultrasonic waves; and
   a surface temperature estimator that estimates the surface temperature based on the temperature difference and the sensed value from the temperature sensing unit (64).

3. The ultrasonic diagnostic device according to claim 2, wherein
   the temperature difference estimation unit estimates the temperature difference based on a power consumption for wave transmission/reception calculated in accordance with the wave transmission/reception condition of the ultrasonic waves.

4. The ultrasonic diagnostic device according to claim 3, wherein
   the power consumption for wave transmission/reception includes a first power consumption at the electronic circuit (60) and a second power consumption at the oscillator (54).

5. The ultrasonic diagnostic device according to claim 1, wherein
   the temperature sensing unit (64) is disposed, in the vicinity of the electronic circuit (60), on a side of the substrate (58) on which the electronic circuit (60) is disposed.

6. The ultrasonic diagnostic device according to claim 5, wherein
   the temperature sensing unit (64) comprises a plurality of temperature sensors disposed on the substrate (58),
   the temperature estimation unit (28) defines a reference temperature based on a plurality of temperatures sensed by the plurality of temperature sensors and estimates the surface temperature based on the reference temperature.

7. The ultrasonic diagnostic device according to claim 6, wherein
   the plurality of temperature sensors are disposed on at least right and left sides of the electronic circuit on the relay board.

8. The ultrasonic diagnostic device according to claim 6, wherein
the temperature estimation unit (28) defines, as the reference temperature, a highest temperature among the plurality of temperatures.

9. The ultrasonic diagnostic device according to claim 1, wherein
the ultrasonic probe (10) is a probe to be inserted into a body cavity;
the oscillator (54) is a 2D array oscillator; and
the electronic circuit (60) is a transceiver circuit having a channel reduction function that supplies a plurality of transmission signals to the 2D array oscillator and processes a plurality of reception signals from the 2D array oscillator.

10. The ultrasonic diagnostic device according to claim 1, further comprising a controller (20) that controls the wave transmission/reception condition of the ultrasonic waves to lower the surface temperature of the wave transmission/reception surface based on the surface temperature of the wave transmission/reception surface.

**Patentansprüche**

1. Ultraschall-Diagnosevorrichtung, umfassend:
eine Ultraschallsonde (10), umfassend:

eine Wellen-Sende/Empfangseinheit (70) mit einem Oszillator (54), der Ultraschallwellen sendet und empfängt,
eine Wellen-Sende/Empfangsfläche des Oszillators (54), die so konfiguriert ist, dass sie auf der Seite eines lebenden Subjekts angeordnet werden kann,
eine Relaisplatte, die als starres Substrat (58) ausgebildet ist, und
eine elektronische Schaltung (60), die elektrisch mit dem Oszillator (54) verbunden ist, und
eine Temperaturerfassungseinheit (64), die in der Wellen- Wellen-Sende/Empfangseinheit angeordnet ist;
wobei
das starre Substrat (58) sandwichartig zwischen der elektronischen Schaltung und dem Oszillator (54) angeordnet ist, und die Temperaturerfassungseinheit (64) auf dem starren Substrat (58) angeordnet ist und wobei die Ultraschalldiagnosevorrichtung ferner eine Temperaturschätzeinheit (28) umfasst, die so konfiguriert ist, dass sie für jede von mindestens zwei Wellen-Sende-/Empfangsbedingungen der Ultraschallwellen eine Oberflächentemperatur der Wellen-Sende-/Empfangsoberfläche auf der Basis von

- den Wellen-Sende-/Empfangszustand der Ultraschallwellen, und
- einem von der Temperaturerfassungseinheit (64) erfassten Wert,

wobei die Temperaturschätzeinheit (28) ferner dazu ausgelegt ist, einen primären Verzögerungsfaktor addiert, der eine Zeitdifferenz berücksichtigt, die erforderlich ist, damit die Wärme die Wellen-Sende/Empfangsfläche der Wärmequelle erreicht, wenn die Oberflächentemperatur der Wellen-Sende/Empfangsfläche geschätzt wird.

2. Ultraschall-Diagnosevorrichtung nach Anspruch 1, wobei die Temperaturschätzeinheit (28) umfasst:

eine Temperaturdifferenz-Schätzeinheit, die eine Differenz zwischen der Temperatur an einer Temperaturerfassungsposition der Temperaturerfassungseinheit (64) und der Temperatur der Wellen-Sende/Empfangsfläche auf der Grundlage der Wellen-Sende/Empfangsbedingung der Ultraschallwellen abschätzt; und
einen Oberflächentemperaturschätzer, der die Oberflächentemperatur auf der Grundlage der Temperaturdifferenz und des von der Temperaturerfassungseinheit (64) erfassten Wertes abschätzt.

3. Ultraschall-Diagnosevorrichtung nach Anspruch 2, wobei die Temperaturdifferenz-Schätzeinheit die Temperaturdifferenz abhängig von einer Leistungsaufnahme für Wellenübertragung/Empfang, die in Übereinstimmung mit dem Wellenübertragungs-/Empfangszustand der Ultraschallwellen berechnet wird, abschätzt.

4. Ultraschall-Diagnosevorrichtung nach Anspruch 3, wobei die Leistungsaufnahme für das Senden/Empfangen von Wellen eine erste Leistungsaufnahme an der elektronischen Schaltung (60) und eine zweite Leistungsaufnahme am Oszillator (54) umfasst.

**5.** Ultraschall-Diagnosevorrichtung nach Anspruch 1, wobei die Temperaturerfassungseinheit (64) in der Nähe der elektronischen Schaltung (60) auf einer Seite des Substrats (58) angeordnet ist, auf der die elektronische Schaltung (60) angeordnet ist.

**6.** Ultraschall-Diagnosevorrichtung nach Anspruch 5, wobei die Temperaturerfassungseinheit (64) eine Vielzahl von Temperatursensoren umfasst, die auf dem Substrat (58) angeordnet sind, die Temperaturschätzeinheit (28) eine Referenztemperatur auf der Grundlage einer Vielzahl von Temperaturen definiert, die von der Vielzahl von Temperatursensoren erfasst werden, und die Oberflächentemperatur auf der Grundlage der Referenztemperatur schätzt.

**7.** Ultraschalldiagnosevorrichtung nach Anspruch 6, wobei
die Mehrzahl der Temperatursensoren zumindest auf der rechten und linken Seite der elektronischen Schaltung auf der Relaisplatine angeordnet sind.

**8.** Ultraschall-Diagnosevorrichtung nach Anspruch 6, wobei die Temperaturschätzungseinheit (28) als Referenztemperatur eine höchste Temperatur unter der Vielzahl von Temperaturen definiert.

**9.** Ultraschall-Diagnosevorrichtung nach Anspruch 1, wobei die Ultraschallsonde (10) eine Sonde ist, die in eine Körperhöhle eingeführt wird;
der Oszillator (54) ein 2D-Array-Oszillator ist; und
die elektronische Schaltung (60) eine Sende/Empfangsschaltung mit einer Kanalreduktionsfunktion ist, die eine Vielzahl von Sendesignalen an den 2D-Array-Oszillator liefert und eine Vielzahl von Empfangssignalen von dem 2D-Array-Oszillator verarbeitet.

**10.** Ultraschall-Diagnosevorrichtung nach Anspruch 1, die ferner eine Steuerung (20) umfasst, die den Wellen-Sende-/Empfangszustand der Ultraschallwellen steuert, um die Oberflächentemperatur der Wellen-Sende-/Empfangsfläche auf der Grundlage der Oberflächentemperatur der Wellen-Sende-/Empfangsfläche zu senken.

**Revendications**

**1.** Dispositif de diagnostic à ultrasons comprenant :

une sonde à ultrasons (10) comprenant une unité émetteur-récepteur d'ondes (70) comprenant un oscillateur (54) qui émet et reçoit des ondes ultrasonores, une surface d'émission/réception d'ondes de l'oscillateur (54) conçue pour être disposée sur un côté d'un sujet vivant, une carte relais qui est formée comme un substrat rigide (58), et un circuit électronique (60) connecté électriquement à l'oscillateur (54), et une unité de détection de température (64) disposée dans l'unité émetteur-récepteur d'ondes ;
dans lequel
le substrat rigide (58) est pris en sandwich entre le circuit électronique et l'oscillateur (54), et
l'unité de détection de température (64) est disposée sur le substrat rigide (58) et dans lequel le dispositif de diagnostic à ultrasons comprend en outre une unité d'estimation de température (28) conçue pour estimer, pour chacune d'au moins deux conditions d'émission/réception d'ondes des ondes ultrasonores, une température de surface de la surface d'émission/réception d'ondes sur la base :

• des conditions d'émission/réception des ondes ultrasonores, et
• d'une valeur détectée, détectée par l'unité de détection de température (64)

dans lequel l'unité d'estimation de température (28) est en outre conçue pour ajouter un facteur de retard principal en considération d'une différence de temps nécessaire pour que la chaleur atteigne la surface d'émission/réception d'ondes à partir de la source de chaleur lors de l'estimation de la température de surface de la surface d'émission/réception d'ondes.

**2.** Dispositif de diagnostic à ultrasons selon la revendication 1, dans lequel
l'unité d'estimation de température (28) comprend
une unité d'estimation de différence de température qui estime une différence entre la température à une position de détection de température de l'unité de détection de température (64) et la température de la surface d'émission/réception d'ondes sur la base de la condition d'émission/réception d'ondes des ondes ultrasonores ; et
un estimateur de température de surface qui estime la température de surface sur la base de la différence de température et de la valeur détectée par l'unité de détection de température (64).

3. Dispositif de diagnostic à ultrasons selon la revendication 2, dans lequel
l'unité d'estimation de différence de température estime la différence de température sur la base d'une consommation d'énergie pour rémission/réception d'ondes calculée conformément à la condition d'émission/réception d'ondes des ondes ultrasonores.

4. Dispositif de diagnostic à ultrasons selon la revendication 3, dans lequel
la consommation d'énergie pour l'émission/réception d'ondes comprend une première consommation d'énergie au niveau du circuit électronique (60) et une seconde consommation d'énergie au niveau de l'oscillateur (54).

5. Dispositif de diagnostic à ultrasons selon la revendication 1, dans lequel
l'unité de détection de température (64) est disposée, à proximité du circuit électronique (60), sur un côté du substrat (58) sur lequel est disposé le circuit électronique (60).

6. Dispositif de diagnostic à ultrasons selon la revendication 5, dans lequel
l'unité de détection de température (64) comprend une pluralité de capteurs de température disposés sur le substrat (58),
l'unité d'estimation de température (28) définit une température de référence sur la base d'une pluralité de températures détectées par la pluralité de capteurs de température et estime la température de surface sur la base de la température de référence.

7. Dispositif de diagnostic à ultrasons selon la revendication 6, dans lequel
la pluralité de capteurs de température sont disposés sur au moins les côtés droit et gauche du circuit électronique sur la carte relais.

8. Dispositif de diagnostic à ultrasons selon la revendication 6, dans lequel
l'unité d'estimation de température (28) définit, en tant que température de référence, une température la plus élevée parmi la pluralité de températures.

9. Dispositif de diagnostic à ultrasons selon la revendication 1, dans lequel
la sonde à ultrasons (10) est une sonde à insérer dans une cavité corporelle ;
l'oscillateur (54) est un oscillateur à matrice 2D ; et
le circuit électronique (60) est un circuit émetteur-récepteur ayant une fonction de réduction de canal qui fournit une pluralité de signaux d'émission à l'oscillateur à matrice 2D et traite une pluralité de signaux de réception provenant de l'oscillateur à matrice 2D.

10. Dispositif de diagnostic à ultrasons selon la revendication 1, comprenant en outre un contrôleur (20) qui commande la condition d'émission/réception d'ondes des ondes ultrasonores pour abaisser la température de surface de la surface d'émission/réception d'ondes sur la base de la température de surface de la surface d'émission/réception d'ondes.

EP 3 097 861 B1

**FIG. 1**

- 18 — DEVICE BODY
- 32 — TRANSCEIVER
- 10 — PROBE
  - 14 — ELECTRONIC CIRCUIT
  - 16 — TEMPERATURE SENSING UNIT
  - 12 — 2D ARRAY OSCILLATOR
  - B, V
- 20 — CONTROLLER
  - 24 — WAVE TRANSMISSION/RECEPTION CONTROLLER
  - 22 — WAVE TRANSMISSION/RECEPTION CONDITION SETTER
  - 26 — POWER CONSUMPTION CALCULATOR
  - 28 — SURFACE TEMPERATURE ESTIMATOR
  - 30 — WARNING CONTROLLER
- 38 — OPERATION PANEL
- 34 — DISPLAY PROCESSOR
- 36 — DISPLAY

FIG. 2

EP 3 097 861 B1

FIG. 3A

60

58

64a

64b

80

82

FIG. 3B

60

58

64

FIG. 3C

60

58

64

90

94

$R_{a1}$

$T_2$ WAVE TRANSMISSION/
RECEPTION SURFACE

92  60

$R_{b1}$  $R_{b2}$

92

$R_{c1}$  $T_1$ TEMPERATURE
SENSOR

FIG. 4

START

S10 — SELECT HEAT
GENERATION MODE

S12 — SET TRANSMISSION
CONDITION

S14 — CALCULATE POWER
CONSUMPTION

S16 — OBTAIN TEMPERATURE SENSOR
SENSED TEMPERATURE $T_1$

S18 — ESTIMATE SURFACE TEMPERATURE
$T_2$ OF WAVE
TRANSMISSION/RECEPTION
SURFACE

S20 — $T_2$

$T_2 < 41°C$

$T_2 = 43°C$

$41°C \leqq T_2 < 43°C$

S22 — DISPLAY WARNING
MESSAGE

S24 — STOP WAVE
TRANSMISSION/RECEPTION

END

FIG. 5

FIG. 6

EP 3 097 861 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2120226160 A1
- US 20140005546 A1
- JP 2013052693 A
- JP 2009034386 A
- JP 2011188956 A
- JP 2009005994 A